# EUROPEAN PATENT APPLICATION

(11) **EP 4 785 888 A1**
(43) Date of publication of application: **05.08.2026**
(21) Application number: 24872801.6
(22) Date of filing: 12.09.2024
(51) Int. Cl.: A61B 34/00, A61B 90/00, A61B 18/20, A61B 18/00

(54) **METHOD FOR PROVIDING AR IMAGE FOR RECOMMENDATION INFORMATION RELATED TO LASER TREATMENT, AND AR WEARABLE DEVICE USING SAME**

(30) Priority: 26.09.2023 KR 20230128929
(71) Applicant: MEDITHINQ CO., LTD, Gyeonggi-do 13486 (KR)
(72) Inventor: JUNG, Yoon Sang, Seongnam-si Gyeonggi-do 13385 (KR)
(74) Representative: Manasse, Uwe
(86) International application number: PCT/KR2024/013894
(87) International publication number: WO 2025/071093

(57) **Abstract**

According to the present invention, there is provided an AR wearable device using an AR image for recommendation information related to laser treatment, comprising: an AR image processing unit configured to receive a video image including a laser treatment area of a patient, divide the laser treatment area into a plurality of mesh regions, and determine display information for each of the plurality of mesh regions; and a display unit for providing the display information for the plurality of mesh regions generated through the AR image processing unit, wherein the display information for each of the plurality of mesh regions further includes recommendation information related to an irradiation order determined based on laser energy information and current laser irradiation position information.

## Description

### Technical Field

The present invention relates to a method for providing an AR image for recommendation information related to laser treatment, and an AR wearable device using the same. More specifically, it relates to a method, device, and system for providing an AR image capable of dividing a laser treatment area of a patient into a plurality of mesh regions and displaying recommendation information related to the laser treatment through an AR image.

### Background Art

Today, various laser treatments and procedural techniques are being developed in which laser beams are irradiated onto the skin for therapeutic purposes, and medical laser devices for such laser treatments are also being actively researched.

A laser beam refers to "special light" produced through a stage of stimulated emission amplification after energy is input into a specific material. Laser treatment is widely used as a convenient method that can be simply performed in a short time without noticeable scarring, as it penetrates this light deep into the skin in an extremely short time to selectively destroy only the targeted skin lesions without damaging surrounding tissues.

Conventionally, such laser treatment devices have been operated manually by practitioners such as doctors during treatment. Accordingly, because the treatment proceeded depending on the subjective experience or senses of the practitioner, there were cases where the precision or efficiency of the laser treatment was degraded. Furthermore, safety issues for the patient arose due to environments where it was difficult for the practitioner to maintain concentration over a prolonged treatment session.

Accordingly, there is a need for a novel laser treatment method and device that can improve the precision or efficiency of laser treatment and ensure the safety of the patient.

### Detailed Description of the Invention

### Technical Problem

An object of the present invention is to provide a method and device for providing an AR image that can improve the precision or accuracy of laser treatment.

Another object of the present invention is to provide a customized AR image providing method and device for a patient to provide an optimal laser treatment method according to the skin characteristics of the patient.

Another object of the present invention is to provide a novel laser treatment method and device capable of improving the precision or accuracy of laser treatment by dividing a laser treatment area of a patient into a plurality of mesh regions and intuitively showing a laser irradiation dose, an alarm display, etc., for each mesh region through color or contrast via an AR image.

Another object of the present invention is to provide a laser treatment method optimized for each patient by providing customized AR images according to the skin characteristics of the patient.

Another object of the present invention is to provide a method and device that allow a practitioner to perform laser treatment more safely and systematically by providing real-time temperature information and laser irradiation dose information of each mesh region within the laser treatment area of the patient through an AR image.

Another object of the present invention is to provide a laser treatment method optimized for the current skin recovery state of a patient by providing recommendation information related to a laser irradiation area order in linkage with laser irradiation history information of a specific skin part at a past specific time point.

The problems to be solved by the present invention are not limited to those mentioned above, and other unmentioned technical problems will be clearly understood by those skilled in the art from the following description.

### Solution for Problem

According to an embodiment of the present invention, there may be provided an AR wearable device using an AR image for recommendation information related to laser treatment, comprising: an AR image processing unit configured to receive a video image including a laser treatment area of a patient, divide the laser treatment area into a plurality of mesh regions, and determine display information for each of the plurality of mesh regions; and a display unit for providing the display information for the plurality of mesh regions generated through the AR image processing unit, wherein the display information for each of the plurality of mesh regions includes recommendation information related to an irradiation order determined based on laser energy information and current laser irradiation position information.

Here, the display information may further include color or contrast information.

In addition, the display information may be set differently based on a skin characteristic value of the patient.

In addition, the display information may be changed based on the amount of time elapsed from a time point when laser irradiation was applied to the corresponding mesh region.

In addition, the recommendation information may be determined based on laser irradiation history information of a specific skin part of the patient at a past specific time point.

In addition, the laser irradiation history information may include information related to time elapsed from the past specific time point, a laser irradiation position, the number of laser irradiations, and a laser irradiation wavelength.

In addition, the AR wearable device may further include a communication unit configured to receive information related to the number of laser irradiations and irradiation energy applied to the patient from a laser irradiator, and the AR image processing unit may be configured to determine recommendation information for each of the plurality of mesh regions based on the number of laser irradiations and irradiation energy associated with each of the plurality of mesh regions.

In addition, color or contrast information of each mesh region may be changed in real-time based on the number of laser irradiations and irradiation energy applied to each of the plurality of mesh regions.

In addition, the AR image processing unit may be configured to calculate an accumulated energy value of each mesh region based on the number of laser irradiations and irradiation energy applied to each of the plurality of mesh regions, and determine whether to generate an alarm display based on whether the accumulated energy value exceeds a preset reference.

In addition, the preset reference compared with the accumulated energy value may be changed based on the skin characteristic value of the patient.

In addition, a size of each of the divided plurality of mesh regions may be changed based on the skin characteristic value of the patient.

In addition, the AR wearable device may further include a temperature sensing sensor configured to acquire real-time temperature information for the laser treatment area of the patient, and the AR image processing unit may process the display information for each of the plurality of mesh regions to further include skin temperature information corresponding to the corresponding mesh region acquired by the temperature sensing sensor.

In addition, the AR image processing unit may be configured to determine whether to generate an alarm display based on whether the skin temperature information exceeds a preset reference.

In addition, according to another embodiment of the present invention, there may be provided a method for providing an AR image for laser treatment using an AR wearable device, comprising the steps of: receiving, through an AR image processing unit, a video image including a laser treatment area of a patient; dividing, through the AR image processing unit, the laser treatment area into a plurality of mesh regions, and determining display information for each of the plurality of mesh regions; and providing, through a display unit, the display information for the plurality of mesh regions generated through the AR image processing unit, wherein the display information for each of the plurality of mesh regions includes recommendation information related to an irradiation order determined based on laser energy information and current laser irradiation position information.

In addition, according to another embodiment of the present invention, there may be provided a computer-readable recording medium storing a computer program for performing the method for providing an AR image described above.

### Effects of the Invention

According to the present invention, a method and device for providing an AR image capable of improving the precision or accuracy of laser treatment can be provided.

In addition, according to the present invention, a customized AR image providing method and device for a patient can be provided to provide an optimal laser treatment method according to the skin characteristics of the patient.

In addition, according to the present invention, a novel laser treatment method and device capable of improving the precision or accuracy of laser treatment can be provided by dividing the laser treatment area of a patient into a plurality of mesh regions and intuitively showing a laser irradiation dose, an alarm display, etc., for each mesh region through color or contrast via an AR image.

In addition, according to the present invention, a laser treatment method optimized for each patient can be provided by providing customized AR images according to the skin characteristics of the patient.

In addition, according to the present invention, a method and device can be provided that allow a practitioner to perform laser treatment more safely and systematically by providing real-time temperature information and laser irradiation dose information of each mesh region within the laser treatment area of the patient through an AR image.

In addition, according to the present invention, a laser treatment method optimized for the current skin recovery state of a patient can be provided by providing recommendation information related to a laser irradiation area order in linkage with laser irradiation history information of a specific skin part at a past specific time point.

The effects of the present invention are not limited to those mentioned above, and other unmentioned effects will be clearly understood by those skilled in the art from the following description.

### Brief Description of Drawings

FIG. 1 is an exemplary view illustrating a state of performing a laser treatment on a patient using an AR wearable device according to an embodiment of the present invention.
FIG. 2 is a block diagram for explaining the configuration of an AR wearable device according to an embodiment of the present invention.
FIG. 3 is a block diagram for explaining the configuration of an AR image processing unit of an AR wearable device according to an embodiment of the present invention.
FIG. 4 is an exemplary view illustrating a plurality of mesh regions into which a treatment area is divided according to an embodiment of the present invention.
FIGS. 5A, 5B, and 5C are exemplary views illustrating various display methods of a plurality of mesh regions into which a treatment area is divided according to an embodiment of the present invention.
FIG. 6 is a flowchart for explaining a method of providing an AR image for laser treatment using an AR wearable device according to an embodiment of the present invention.

### Detailed Description for Implementing the Invention

Hereinafter, the present invention will be described in detail with reference to the accompanying drawings so that those skilled in the art to which the present invention pertains can easily carry out the same. However, the present invention may be implemented in various different forms and is not limited to the embodiments described herein.

Terms used in this specification are for the purpose of describing embodiments and are not intended to limit the present invention. In this specification, singular forms also include plural forms unless explicitly stated otherwise in the context.

As used herein, "comprises" and/or "comprising" do not exclude the presence or addition of one or more other components, steps, operations, and/or elements in addition to the mentioned components, steps, operations, and/or elements.

In addition, terms including ordinal numbers such as first and second used in the present invention may be used to describe components, but the components should not be limited by the terms. Such terms are used only for the purpose of distinguishing one component from another. In addition, in describing the present invention, if it is determined that a detailed description of a related known technology may obscure the gist of the present invention, the detailed description thereof will be omitted.

In addition, components shown in the embodiments of the present invention are shown independently to indicate different characteristic functions, and it does not mean that each component consists of separate hardware or a single software component. That is, for convenience of description, each component is listed and described as an individual component, and at least two components of each component may be combined into one component, or one component may be divided into a plurality of components to perform functions. Integrated embodiments and separated embodiments of each of these components are also included in the scope of the present invention without departing from the essence of the present invention.

Hereinafter, embodiments according to the present invention will be described in detail with reference to the accompanying drawings. The configuration of the present invention and its operational effects will be clearly understood through the detailed description below.

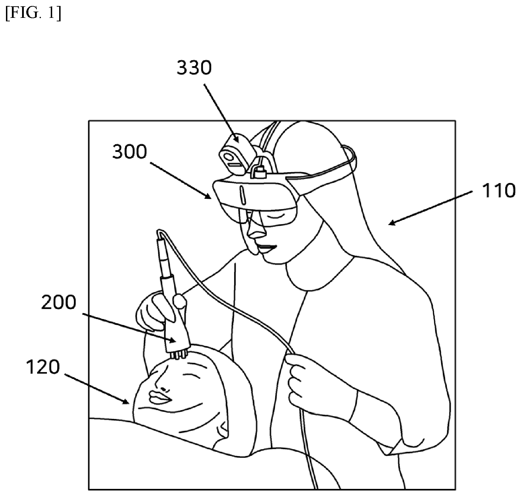

FIG. 1 is an exemplary view illustrating a state of performing a laser treatment on a patient using an AR wearable device according to an embodiment of the present invention.

During laser treatment or procedure, a laser practitioner 110 may wear an Augmented Reality (AR) wearable device 300 according to the present invention and irradiate a laser onto a treatment area of a patient 120 using a laser irradiator 200. The laser practitioner 110 may be medical personnel such as a doctor or a nurse.

The AR wearable device 300 has a form that can be worn on the body such as the head or face of the practitioner, and may be configured to divide the laser treatment area visible to the practitioner 110 into a plurality of mesh regions and display them, and to show display information including color or contrast information together for each of the plurality of mesh regions. A more specific configuration of the AR wearable device 300 will be described in detail below.

The AR wearable device 300 may also include a temperature sensing sensor 330 configured to acquire real-time temperature information about the laser treatment area, and the AR wearable device 300 may provide skin temperature information corresponding to the corresponding mesh region acquired by the temperature sensing sensor 330 together through display information for each of the plurality of mesh regions.

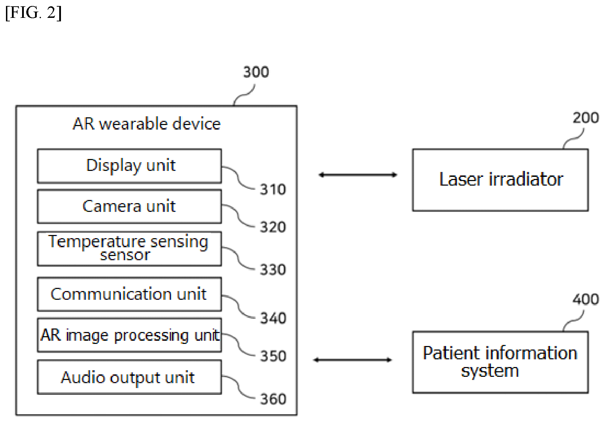

FIG. 2 is a block diagram for explaining the configuration of an AR wearable device according to an embodiment of the present invention.

The AR wearable device 300 has a form wearable on the head or face of the laser practitioner, is configured to provide visual information for laser treatment through a display to provide an augmented reality service, and is configured to include a display unit 310, a camera unit 320, a temperature sensing sensor 330, a communication unit 340, an AR image processing unit 350, and an audio output unit 360, but is not limited to these components.

The display unit 310 is configured to display visual information to the practitioner 110 wearing the AR wearable device 300. For example, the display unit 310 may use at least one of a half-reflective curved mirror method capable of optical see-through, a light guide method, or a waveguide method, or a method of providing a Mixed Reality image obtained by adding necessary information to a gaze image obtained through the camera unit 320, thereby providing the practitioner 110 with an augmented reality experience. In particular, the display unit 310 may be configured to show a state in which the laser treatment area generated through the AR image processing unit 350 is divided into a plurality of mesh regions, and display information including laser irradiation information and skin temperature information expressed as color or contrast information for each of the plurality of mesh regions, together with reality information.

The camera unit 320 is configured to acquire a captured image by photographing an image in front of the practitioner 110, and may include, for example, an RGB-Depth camera capable of acquiring RGB images and depth information, or as another example, an RGB acquisition module capable of acquiring RGB images and a depth acquisition module capable of acquiring depth information. After acquiring the captured RGB image and depth information through the camera unit 320, a 3D position of at least one object included in the image may be acquired based on a camera coordinate system. The video image acquired through the camera unit 320 may be transmitted to the AR image processing unit 350 for AR image processing.

The temperature sensing sensor 330 is mounted on the AR wearable device 300 and is configured to detect the skin temperature of the patient 120. For example, it may be a non-contact temperature sensor, such as a thermal imaging camera that detects temperature with infrared rays, and may measure skin temperature through the amount of radiation emitted from the skin surface. For example, the temperature sensing sensor 330 may be manufactured through a CMOS process and may be a sensor capable of detecting the temperature of a camera image pixel-by-pixel. The thermal image and skin temperature information obtained through the temperature sensing sensor 330 may be transmitted to the AR image processing unit 350 for AR image processing.

The communication unit 340 may include one or more wired/wireless communication modules for communication with external devices, for example, a shortrange communication unit and a mobile communication unit, and may be configured to transmit and receive necessary data to and from the laser irradiator 200 and a patient information system 400 through wired/wireless communication, etc. Here, the shortrange wireless communication unit may include, for example, a Bluetooth communication unit, a BLE (Bluetooth Low Energy) communication unit, a Near Field Communication unit, a WLAN (Wi-Fi) communication unit, a Zigbee communication unit, an IrDA (Infrared Data Association) communication unit, a WFD (Wi-Fi Direct) communication unit, a UWB (Ultra Wideband) communication unit, an Ant+ communication unit, etc., but is not limited thereto. In addition, the mobile communication unit may transmit and receive wireless signals with at least one of a base station, an external terminal, or a server over a mobile communication network, and the wireless signals may include various types of data resulting from the transmission and reception of, for example, voice call signals, video call signals, or text/multimedia messages.

The AR wearable device 300 may be connected to the laser irradiator 200 through wired/wireless communication via the communication unit 340 to receive setting information or irradiation information of the laser irradiator 200, and may receive laser irradiation-related setting information in real-time, such as the number of laser irradiations (number of operation times of a laser shot), irradiation energy per laser shot, and laser wavelength information. Furthermore, the AR wearable device 300 may receive previous laser treatment information and skin characteristic value information of the corresponding patient through the patient information system 400. Here, the previous laser treatment information may include previous treatment area position information, laser treatment type information, number of laser shot irradiations, and energy information, and the skin characteristic value information of the patient may include skin sensitivity information, allergy information, and information such as moisture content, epidermal thickness, and dermal thickness acquired according to skin measurement of the patient. In addition to the skin characteristic value of the patient, external environment factors such as seasonal information considering outside temperature and humidity, the type of moisturizer or cosmetics applied to the skin of the patient, and time information after applying the moisturizer or cosmetics may be additionally considered.

The AR image processing unit 350 may be configured to divide the laser treatment area photographed by the camera unit 320 into a plurality of mesh regions and determine display information for each of the plurality of mesh regions, wherein 3D position information acquired through the camera coordinate system may be used to register the display information to appear on the corresponding mesh region. Here, the mesh region may be composed of points, lines, polygons, polyhedrons, etc., and may be configured, for example, as a polygon mesh divided into a plurality of mesh regions based on similarity of vector values such as a normal vector. Also, the size of the mesh region may be changed as precision is adjusted by adjusting the similarity determination reference of the vector value.

Here, the display information displayed for each mesh region may be configured such that color or contrast information of each mesh region is changed in real-time based on the number of laser irradiations and irradiation energy applied to each of the plurality of mesh regions. For example, based on a value obtained by multiplying the number of laser irradiations by the irradiation energy, an accumulated value of laser energy applied to the corresponding mesh region may be calculated, and based on the accumulated laser energy value, the color or contrast information of the corresponding mesh region may be changed. For example, as the applied energy is larger, the contrast of the corresponding mesh region may be displayed darker, so that the practitioner can intuitively grasp the applied energy value.

In addition, the display information displayed for each mesh region may be set differently based on the skin characteristic value of the patient measured through the camera unit 320 or acquired through the patient information system 400. For example, the AR image processing unit 350 may calculate an accumulated energy value of each mesh region based on the number of laser irradiations and irradiation energy applied to each of the plurality of mesh regions, and may determine whether to generate an alarm display based on whether the accumulated energy value exceeds a preset reference to represent the alarm display as display information, and at this time, the preset reference compared with the accumulated energy value may be changed based on the skin characteristic value of the patient. That is, when the skin characteristic of the patient is sensitive or weak, the alarm display generation reference can be set more sensitively. For example, the contrast display reference at a warning level during laser treatment may be set differently according to the skin characteristic value of the patient, so that when the same laser energy is applied, a patient with strong skin characteristics does not reach the contrast display representing the alarm display, but a patient with weak skin characteristics can quickly reach the contrast display representing the alarm display. Also, for example, when it is determined that the moisture content of the patient's skin is relatively high, the contrast display reference representing the alarm display may be adjusted so that the contrast display reference at the warning level is set relatively less sensitively. In this way, by changing the display information of the mesh region reflecting the skin characteristic value of the patient, customized information optimized for each patient can be provided.

In addition, the AR image processing unit 350 may be configured to change the size of each of the divided plurality of mesh regions based on the skin characteristic value of the patient. For example, when the skin characteristics of the patient are sensitive or weak, the size setting of the mesh region may be set small, and when the skin characteristics of the patient are less sensitive and strong, the size setting of the mesh region may be configured to be large.

In addition, display information such as color or contrast displayed for each mesh region may be changed based on the amount of time elapsed from the time point when laser irradiation was applied to the corresponding mesh region. For example, the skin characteristic value may be corrected by simulating skin recovery at the current time point in consideration of the time elapsed after laser irradiation was applied to each mesh region, and the time required for collagen generation and elastin fiber regeneration for each patient according to a specific type or a specific wavelength band of laser treatment on a specific area, and the display information may be changed based on this corrected skin characteristic value. For example, after displaying the contrast of the corresponding mesh region darkly based on the wavelength or size of the irradiation energy and the number of laser irradiations applied to the corresponding mesh region, the contrast of the corresponding mesh region may be displayed lighter according to the amount of time elapsed after the laser treatment is finished, and such display information values may be updated and stored to be utilized during the next treatment. For example, when an Nd:YAG laser having a wavelength of 1064 nm is used, it can induce collagen generation in the dermal layer to obtain a skin regeneration effect as well as an effect of improving vascular lesions and pigmented lesions in the dermal layer. Alternatively, when a copper vapor laser in which copper vapor is a gain medium is used as a laser generating two wavelengths: a green wavelength of 511 nm and a yellow wavelength of 578.2 nm, it can be used for the treatment of vascular lesions.

In addition, the AR image processing unit 350 may process the display information for each of the plurality of mesh regions to further include skin temperature information corresponding to the corresponding mesh region acquired by the temperature sensing sensor 330, and for example, may display a color corresponding to the skin temperature information or display separate temperature numerical information together on each mesh region. Furthermore, the AR image processing unit 350 may be configured to determine whether to generate an alarm display based on whether the skin temperature information exceeds a preset reference, and such an alarm generation reference for the skin temperature information may be changed based on the skin characteristic value of the patient.

The audio output unit 360 may be configured to output an audio when an alarm display is generated through the AR image processing unit 350. By outputting a warning sound or alarm-related information via audio when a warning (alarm) display that may occur during laser treatment is reached for the practitioner 110 or the patient 120, patient safety functions can be strengthened.

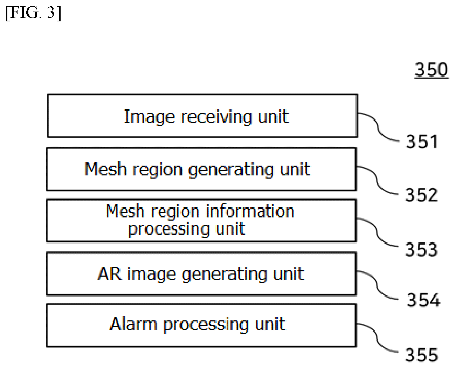

FIG. 3 is a block diagram for explaining the configuration of an AR image processing unit of an AR wearable device according to an embodiment of the present invention.

The AR image processing unit 350 may include an image receiving unit 351, a mesh region generating unit 352, a mesh region information processing unit 353, an AR image generating unit 354, and an alarm processing unit 355. The AR image processing unit 350 may include programs or program modules executable by one or more processors and a necessary memory. Here, the programs or program modules may be configured in the form of an operating system, an application program, or a program, and may be physically stored on various types of widely used storage devices. Such programs or program modules may include, but are not limited to, various forms for performing one or more routines, subroutines, programs, objects, components, instructions, data structures, and specific tasks or executing specific data types.

First, the image receiving unit 351 may be configured to receive a video image including a laser treatment area of a patient acquired through the camera unit 320 or a separate camera device. In addition, the image receiving unit 351 may be configured to receive a thermal image acquired through the temperature sensing sensor 330. Furthermore, the image receiving unit 351 may be configured to receive or generate an image in which the thermal image acquired through the temperature sensing sensor 330 and the real image acquired through the camera unit 320 are semi-transparently superimposed on each other.

The mesh region generating unit 352 may be configured to divide the laser treatment area into a plurality of mesh regions to generate a plurality of divided regions such as polygons. The mesh region generating unit 352 may perform an image analysis operation that isolates only the treatment area by analyzing the video image acquired through the image receiving unit 351. For example, by analyzing an image of the patient, if the treatment area is a face, the facial area alone may be separated as an object, and the facial area alone may be divided into a plurality of mesh regions to generate mesh regions only for the corresponding treatment area. In addition, the mesh region generating unit 352 may be configured to change the size of each of the divided plurality of mesh regions based on the skin characteristic value of the patient when determining it.

The mesh region information processing unit 353 may be configured to generate display information added as an AR image. The mesh region information processing unit 353 may determine display information for each of the generated plurality of mesh regions, and the display information may include color or contrast information so that the colors or contrasts of the respective mesh regions are displayed differently. Here, the display information may be set differently by applying different references for each individual even for the same numerical value based on the skin characteristic value of the patient. In addition, the display information may be set to be changed based on the amount of time elapsed from the time point when laser irradiation was applied to the corresponding mesh region. Further, after receiving information related to the number of laser irradiations and irradiation energy applied to the patient from the laser irradiator 200 through the communication unit 340, the mesh region information processing unit 353 may be configured to calculate an accumulated energy value applied to each mesh region based on the number of laser irradiations and irradiation energy associated with each of the plurality of mesh regions, and based on this, determine the display information for each of the plurality of mesh regions. Also, for example, the mesh region information processing unit 353 may be configured to change the color or contrast information of each mesh region in real-time based on the number of laser irradiations and irradiation energy applied to each of the plurality of mesh regions. According to such change in display information, the practitioner 110 can intuitively and quickly grasp the cumulative laser irradiation dose applied to the corresponding mesh region and proceed with the treatment more systematically.

In addition, the display information of each mesh region may include alarm display information, and the alarm display information may also be displayed in a specific color or contrast or appear as a separate alarm display. For example, the mesh region information processing unit 353 may be configured to calculate an accumulated energy value of each mesh region based on the number of laser irradiations and irradiation energy applied to each of the plurality of mesh regions, and determine whether to generate an alarm display based on whether the accumulated energy value exceeds a preset reference. Here, the preset reference compared with the accumulated energy value may be set to be changed based on the skin characteristic value of the patient, thereby determining whether to generate an alarm display in a manner tailored to the skin characteristics of each patient. Moreover, an alarm display may be set to be generated when the accumulated energy value of each of a predetermined number or more of adjacent mesh regions exceeds a preset reference.

In addition, the mesh region information processing unit 353 may be configured to further generate, as display information, recommendation information related to an irradiation order for an optimal laser treatment effect based on the laser energy information and the current laser irradiation position information, and accordingly, the practitioner 110 can conveniently identify the optimal position and order of the laser irradiation area through the AR image generated on the display unit 310. Furthermore, recommendation information regarding the irradiation order of the optimal laser irradiation area may be provided by reflecting the current skin recovery capacity for each patient, such as causing a mild burn in the area and regenerating it by lasers of different wavelengths irradiated on a specific area of the patient in the past, or temporally simulating collagen generation and elastin fiber regeneration in the dermal layer and subcutaneous fascia layer of the corresponding area. For this purpose, the recommendation information may be determined based on laser irradiation history information of a specific skin part of each patient at a past specific time point, and the laser irradiation history information may include, for example, information related to the time elapsed from a past specific time point, a laser irradiation position related to a past laser treatment, the number of laser irradiations, and a laser irradiation wavelength. Additionally, the recommendation information related to laser irradiation may be updated by reflecting the relationship between whether treatment was performed according to the recommended laser irradiation order and the resulting patient skin prognosis information later.

In addition, the display information of each mesh region may further include skin temperature information corresponding to the corresponding mesh region acquired by the temperature sensing sensor 330, and the skin temperature information may be displayed as numbers, colors, or the like. In addition, the mesh region information processing unit 353 may be configured to determine whether to generate an alarm display based on whether the skin temperature information exceeds a preset reference according to each patient.

The AR image generating unit 354 is configured to generate an AR image by adding the plurality of mesh regions generated through the mesh region generating unit 352 and the display information for each of the mesh regions through the mesh region information processing unit 353, and the generated AR image may be displayed through the display unit 310 via an optical see-through method or via a method of providing a Mixed Reality image obtained by adding AR image information to a gaze image acquired through the camera 320.

The alarm processing unit 355 is configured to provide an additional alarm when an alarm display is generated by the mesh region information processing unit 353, and for example, may be configured to output audio announcing the alarm through the audio output unit 360.

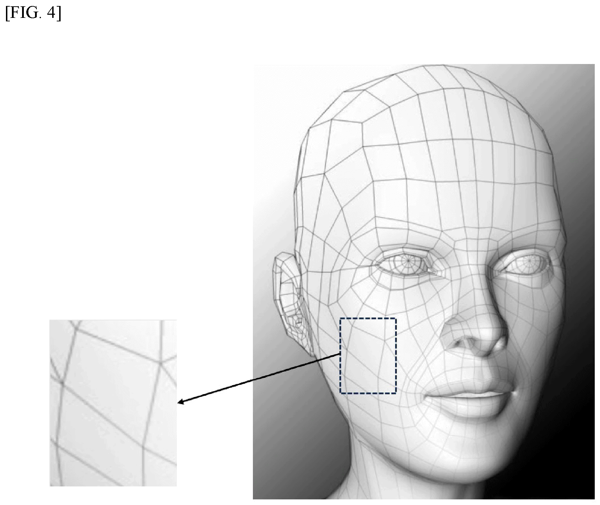

FIG. 4 is an exemplary view illustrating a plurality of mesh regions into which a treatment area is divided according to an embodiment of the present invention.

Referring to FIG. 4, when the laser treatment area of the patient 120 is, for example, a face and a neck, a face and neck area may be recognized from a captured image of the patient, and the treatment area may be divided into a plurality of polygon-shaped mesh regions. A division method may divide the area into a plurality of mesh regions based on a similarity of a vector value such as a normal vector, and various other methods may be used.

Here, color or contrast may be displayed differently for each divided mesh region, and for example, color or contrast may be determined in real-time based on an accumulated energy value calculated based on the number of laser irradiations and irradiation energy, and color or contrast may be changed reflecting a skin recovery capacity based on the amount of time elapsed from the time point when laser irradiation was applied. In addition, color or contrast may be changed reflecting the degree of skin recovery at the current time point for each patient, such as causing a mild burn in the area and regenerating it by lasers of different wavelengths irradiated on a specific area in the past, or temporally simulating collagen generation and elastin fiber regeneration in the dermal layer and subcutaneous fascia layer of the area. Further, during laser treatment, when the accumulated energy value exceeds a preset reference, a specific color or contrast for displaying an alarm for the corresponding mesh region may be displayed, and a determination reference for determining whether to display such an alarm may be applied differently according to the skin characteristic value of the patient.

In addition, the size of each mesh region may be changed according to the skin characteristic value of the patient by adjusting the similarity determination reference of the vector value.

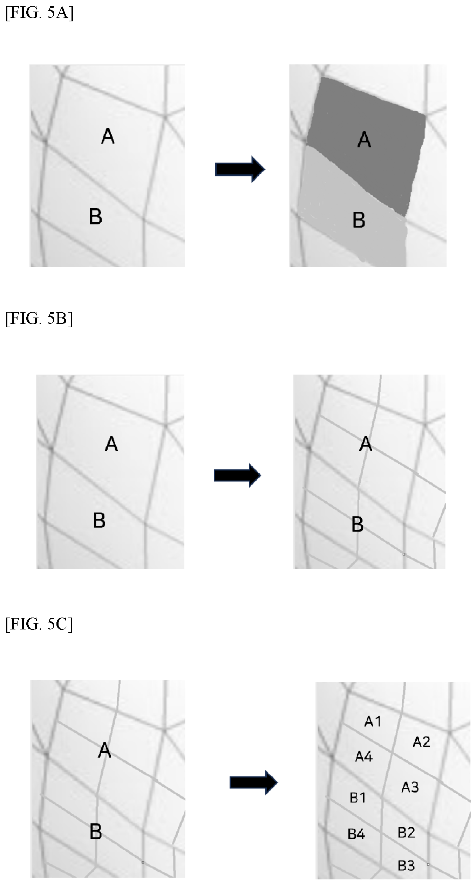

FIGS. 5A, 5B, and 5C are exemplary views illustrating various display methods of a plurality of mesh regions into which a treatment area is divided according to an embodiment of the present invention.

Referring to FIG. 5A, it can be seen that the contrast of each mesh region is changed based on the accumulated energy value applied to the corresponding mesh region during laser treatment. For example, when the accumulated energy value applied to a mesh region A is greater than the accumulated energy value applied to a mesh region B, the contrast of the mesh region A may be displayed darker than the contrast of the mesh region B, and the practitioner 110 can quickly determine the degree of energy applied to each mesh region through the displayed contrast information.

Referring to FIG. 5B, the size of each mesh region may be changed according to the skin characteristic value of the patient. For example, when the skin characteristics of the patient have high sensitivity or represent weak skin, the size of the mesh region is set finer and smaller as shown in the right drawing, thereby providing display information related to laser treatment for a more precise range.

Referring to FIG. 5C, information related to an irradiation order determined based on laser energy information and current laser irradiation position information may be provided for each mesh region. For example, in a mesh region A divided into four parts, an irradiation order is determined as A1, A2, A3, and A4 based on the degree of proximity to the current laser irradiation position and the similarity of the magnitude information of the laser energy to be irradiated to the corresponding mesh region. Next, in a mesh region B divided into four parts adjacent to the mesh region A, an irradiation order is similarly determined as B1, B2, B3, and B4. By displaying this as display information, it can assist the practitioner 110 in systematically determining the irradiation position and order during laser irradiation.

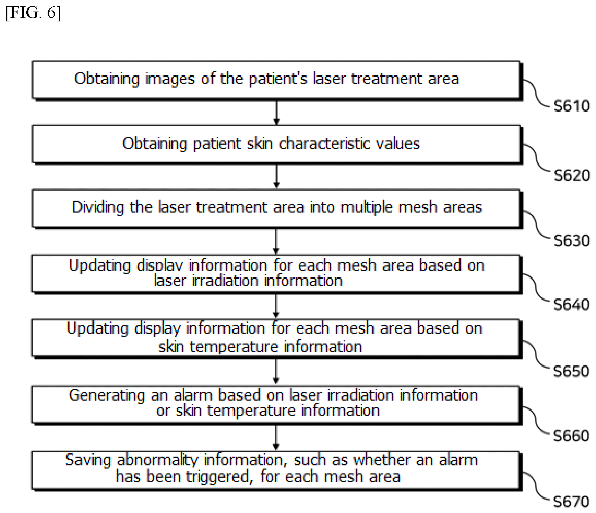

FIG. 6 is a flowchart for explaining a method of providing an AR image for laser treatment using an AR wearable device according to an embodiment of the present invention.

Referring to FIG. 6, the AR wearable device 300 may acquire image data photographing the laser treatment area of the patient through the camera unit 320. (S610)

In addition, the AR wearable device 300 may acquire a skin characteristic value of the patient, such as skin sensitivity information, allergy information, and information such as moisture content, epidermal thickness, and dermal thickness acquired according to skin measurement of the patient, either through photographing the skin by the camera unit 320 or from those stored in the patient information system 400. (S620)

The AR wearable device 300 may divide the laser treatment area into a plurality of mesh regions through the AR image processing unit 350. (S630)

The AR wearable device 300 may update display information for each of the mesh regions based on laser irradiation information for each mesh region through the AR image processing unit 350. (S640) For example, the color or contrast of each mesh region may be changed in real-time based on an accumulated laser energy value calculated using the number of laser irradiations and irradiation energy for each mesh region.

The AR wearable device 300 may update display information for each of the mesh regions based on skin temperature information through the AR image processing unit 350. (S650) Based on the skin temperature information of each mesh region acquired through the temperature sensing sensor 330, the color or contrast of each mesh region may be changed in real-time, or the skin temperature information may be expressed as a numerical value.

The AR wearable device 300 may generate an alarm display based on the laser irradiation information or the skin temperature information. (S660) The determination reference for determining whether to generate such an alarm display may be applied differently according to the skin characteristic value of the patient, and may be configured to generate an alarm display when the accumulated energy value through laser irradiation or the skin temperature information deviates from a predetermined determination reference.

The AR wearable device 300 may store abnormality information, such as whether an alarm is generated for each mesh region. (S670) Based on alarm information generated during laser treatment for the corresponding patient, information on the corresponding mesh region and information related to the cause of alarm generation are stored, and by providing such information during the next laser treatment, the laser treatment can be carried out more safely.

## Claims

1. An AR wearable device using an AR image for recommendation information related to laser treatment, comprising:
an AR image processing unit configured to receive a video image including a laser treatment area of a patient, divide the laser treatment area into a plurality of mesh regions, and determine display information for each of the plurality of mesh regions; and
a display unit for providing the display information for the plurality of mesh regions generated through the AR image processing unit,
wherein the display information for each of the plurality of mesh regions includes recommendation information related to an irradiation order determined based on laser energy information and current laser irradiation position information,
wherein the display information is set differently based on a skin characteristic value of the patient,
wherein the display information is changed based on an amount of time elapsed from a time point when laser irradiation was applied to the corresponding mesh region,
wherein the skin characteristic value of the patient is corrected based on a result of simulating a skin recovery capacity at a current time point using information related to a laser irradiation wavelength applied to each mesh region of the patient and the amount of time elapsed from a time point when the laser irradiation was applied,
wherein the recommendation information is determined based on laser irradiation history information of a specific skin part of the patient at a past specific time point, and wherein
the laser irradiation history information includes the result of simulating the skin recovery capacity at the current time point.

2. The AR wearable device of claim 1, wherein the display information further includes color or contrast information.

3. The AR wearable device of claim 1, wherein the laser irradiation history information includes information related to time elapsed from the past specific time point, a laser irradiation position, the number of laser irradiations, and a laser irradiation wavelength.

4. The AR wearable device of claim 1, further comprising
a communication unit configured to receive information related to the number of laser irradiations and irradiation energy applied to the patient from a laser irradiator,
wherein the AR image processing unit is configured to determine recommendation information for each of the plurality of mesh regions based on the number of laser irradiations and irradiation energy associated with each of the plurality of mesh regions.

5. The AR wearable device of claim 1, wherein color or contrast information of each mesh region is changed in real-time based on the number of laser irradiations and irradiation energy applied to each of the plurality of mesh regions.

6. The AR wearable device of claim 1, wherein the AR image processing unit is configured to calculate an accumulated energy value of each mesh region based on the number of laser irradiations and irradiation energy applied to each of the plurality of mesh regions, and determine whether to generate an alarm display based on whether the accumulated energy value exceeds a preset reference.

7. The AR wearable device of claim 6, wherein the preset reference compared with the accumulated energy value is changed based on the skin characteristic value of the patient.

8. The AR wearable device of claim 1, wherein a size of each of the divided plurality of mesh regions is changed based on the skin characteristic value of the patient.

9. The AR wearable device of claim 1, further comprising
a temperature sensing sensor configured to acquire real-time temperature information for the laser treatment area of the patient,
wherein the AR image processing unit further includes skin temperature information corresponding to the corresponding mesh region acquired by the temperature sensing sensor in the display information for each of the plurality of mesh regions.

10. The AR wearable device of claim 9, wherein the AR image processing unit is configured to determine whether to generate an alarm display based on whether the skin temperature information exceeds a preset reference.

11. A method for providing an AR image for laser treatment using an AR wearable device, comprising the steps of:
receiving, through an AR image processing unit, a video image including a laser treatment area of a patient;
dividing, through the AR image processing unit, the laser treatment area into a plurality of mesh regions, and determining display information for each of the plurality of mesh regions; and
providing, through a display unit, the display information for the plurality of mesh regions generated through the AR image processing unit,
wherein the display information for each of the plurality of mesh regions includes recommendation information related to an irradiation order determined based on laser energy information and current laser irradiation position information,
wherein the display information is set differently based on a skin characteristic value of the patient, wherein the display information is changed based on an amount of time elapsed from a time point when laser irradiation was applied to the corresponding mesh region, wherein the skin characteristic value of the patient is corrected based on a result of simulating a skin recovery capacity at a current time point using information related to a laser irradiation wavelength applied to each mesh region of the patient and the amount of time elapsed from a time point when the laser irradiation was applied,
wherein the recommendation information is determined based on laser irradiation history information of a specific skin part of the patient at a past specific time point, and
wherein the laser irradiation history information includes the result of simulating the skin recovery capacity at the current time point.

12. A computer-readable recording medium storing a computer program for performing the method according to claim 11.
